# EUROPEAN PATENT SPECIFICATION

(11) **EP 1 964 505 B1**
(45) Date of publication and mention of the grant of the patent: **03.06.2015**
(21) Application number: 06834955.4
(22) Date of filing: 19.12.2006
(51) Int. Cl.: A61B 1/00, A61B 5/07, G02B 23/24, B29C 65/16

(54) **CAPSULE ENDOSCOPE AND MANUFACTURING METHOD THEREOF**
KAPSEL-ENDOSKOP UND VERFAHREN ZU SEINER HERSTELLUNG
ENDOSCOPE À CAPSULE ET PROCÉDÉ POUR FABRIQUER CELUI-CI

(30) Priority: 19.12.2005 JP 2005365209
(43) Date of publication of application: 03.09.2008
(73) Proprietor: OLYMPUS MEDICAL SYSTEMS CORP., Tokyo 151-0072 (JP)
(72) Inventor: OKUZUMI, Koji c/o Intellectual Property Support Department, Tokyo 192-8512 (JP); MATSUMOTO, Jun c/o Intellectual Property Support Department, Tokyo 192-8512 (JP); NAKAMURA, Takeaki c/o Intellectual Property Support Department, Tokyo 192-8512 (JP)
(74) Representative: von Hellfeld, Axel
(86) International application number: PCT/JP2006/325245
(87) International publication number: WO 2007/072808

(56) References cited:
- EP-A1- 1 523 933
- WO-A1-03/011574
- JP-A- 09 173 477
- JP-A- 2005 087 295
- JP-A- 2005 261 504
- US-B1- 6 547 722

## Description

### Technical Field

The present invention relates to a small-sized capsule endoscope formed into a capsule-shape used for inspection or the like inside a living body, and a method of manufacturing the same.

### Background Art

Techniques related to capsule endoscopes are disclosed in, for example, Jpn. Pat. Appln. KOKAI Publication No. 2001-91860 (paragraph No. [0012]), Jpn. Pat. Appln. KOKAI Publication No. 2004-65575 (paragraph Nos. [0028], [0071]), and Jpn. Pat. Appln. KOKAI Publication No. 2005-261504 (paragraph No. [0033]). In Jpn. Pat. Appln. KOKAI Publication No. 2001-91860, that a capsule endoscope includes an exterior case in which a circuit board and the like are encased in a watertight state, the exterior case is constituted of a substantially hemispheric transparent cover covering the front of an objective lens, and a cylindrical cover covering the rear of the objective lens and having a hemispheric shape at a rear end thereof, and the transparent cover and the cylindrical cover are bonded to each other in a watertight state, thereby forming a capsule endoscope is disclosed.

In Jpn. Pat. Appln. KOKAI Publication No. 2004-65575, that a capsule endoscope includes a capsule container sealed by using a front-end cover and a rear-end cover, an O-ring for watertight sealing is interposed between an outer circumferential surface of the main body and an inner circumferential surface of the rear cover, and the front-end cover constituted of a flexible material and the main body are fixed to each other by using an adhesive that can secure watertightness even when it is subjected to elastic deformation is disclosed.

In Jpn. Pat. Appln. KOKAI Publication No. 2005-261504, that an observation side cover and a capsule main body are fixed to each other by welding so as to be integral with each other by means of ultrasonic heat generation achieved by applying ultrasonic vibration to the cover and the main body is disclosed.
Pat. Document 1: Jpn. Pat. Appln. KOKAI Publication No. 2001-91860 (paragraph No. [0012])
Pat. Document 2: Jpn. Pat. Appln. KOKAI Publication No. 2004-65575 (paragraph No. [0028], [0071])
Pat. Document 3: Jpn. Pat. Appln. KOKAI Publication No. 2005-261504 (paragraph No. [0033])
JP 2005-087295 A discloses an ultrasonic endoscope.

### Disclosure of Invention

In the techniques disclosed in Jpn. Pat. Appln. KOKAI Publication No. 2001-91860 and Jpn. Pat. Appln. KOKAI Publication No. 2004-65575, for example, when the transparent cover and the cylindrical cover are bonded to each other, it is difficult to achieve separation with constant application amount of adhesive. Further, in the technique disclosed in the above patent documents, when the transparent cover and the cylindrical cover are bonded to each other, an excessive amount of adhesive is applied to the outer surface of the capsule main body. An uneven part or a burr-like part is formed by the adhesive on the outer surface of the capsule main body in some cases. If such an uneven part or a burr-like part is formed, according to the above patent documents, complicated post-treatment process work must be performed in order to finish the outer surface of the capsule main body smooth.

The capsule endoscope is swallowed into a body of a person such as a patient, is used to inspect the inside of the living body, and is small-sized. It is undesirable for such a small-sized capsule endoscope that an uneven part or a burr-like part is formed at, for example, a bonded part at which the transparent cover and the cylindrical cover are bonded to each other. It is therefore necessary for the small-sized capsule endoscope that the bonding should be reliably performed with the outer surface of the capsule main body finished smooth.

The technique disclosed in Jpn. Pat. Appln. KOKAI Publication No. 2005-261504 can solve the problems of the above patent documents. However, in the technique of Jpn. Pat. Appln. KOKAI Publication No. 2005-261504, an ultrasonic horn is brought into contact with the observation side cover and the capsule main body, and an ultrasonic wave is oscillated. Therefore, there is the possibility of electronic components and the like contained in the capsule main body being adversely affected.

In the technique of Jpn. Pat. Appln. KOKAI Publication No. 2005-261504, the ultrasonic horn is brought into contact with the cover and the capsule main body, and hence a surface of a part of the resin which is brought into contact with the horn is heated. Thus, by the technique of the above patent document, the surface is roughened, and no smooth capsule outer surface can be obtained. Further, using the technique of the above patent document, it is difficult to achieve, for example, narrow and linear adhesion in the ultrasonic welding.

Besides the above problem, in order to bring the ultrasonic horn into contact with the observation side cover or the capsule main body, and effectively transmit the ultrasonic wave, there is the necessity to provide a notch or the like in the part of the cover or the capsule main body at which the ultrasonic horn is brought into contact with the cover or the capsule main body.

An object of the present invention is to provide a capsule endoscope in which welding and bonding can be securely performed by irradiation of laser light, and a method of manufacturing the capsule endoscope.

According to an example useful for understanding present invention, there is provided a capsule endoscope comprising: a first exterior member formed of an optical resin member transmitting visible light and laser light; and a second exterior member which is arranged with respect to the first exterior member in such a manner that the second exterior member is brought into surface contact with the first exterior member, and is formed of a resin member containing a laser light absorbing agent, characterized in that laser light is applied to the second exterior member through the first exterior member, thereby melting the second exterior member, and the first exterior member and the second exterior member are bonded to each other by laser welding.

According to the present invention, a capsule endoscope comprises: a first exterior member formed of an optical resin member transmitting visible light and laser light; a second exterior member which is arranged with respect to the first exterior member in such a manner that the second exterior member is brought into surface contact with the first exterior member, and is formed of a resin member; and a laser light absorbing member provided at a part at which the first exterior member and the second exterior member are in surface contact with each other, wherein laser light is applied to the laser light absorbing member through the first exterior member, thereby melting the laser light absorbing member, and the first exterior member and the second exterior member are bonded to each other by laser welding.

According to an example useful for understanding the present invention, a method of manufacturing a capsule endoscope comprises: arranging a second exterior member formed of a resin member containing a laser light absorbing agent with respect to a first exterior member formed of an optical resin member transmitting visible light and laser light in such a manner that the second exterior member is brought into surface contact with the first exterior member; and applying laser light to the second exterior member through the first exterior member, thereby melting the second exterior member, and bonding the first exterior member and the second exterior member to each other by laser welding.

According to the present invention, a method of manufacturing a capsule endoscope comprises: arranging a second exterior member formed of a resin member with respect to a first exterior member formed of an optical resin member transmitting visible light and laser light in such a manner that the second exterior member is brought into surface contact with the first exterior member; providing a laser light absorbing member at a part at which the first exterior member and the second exterior member are in surface contact with each other; and applying laser light to the laser light absorbing member through the first exterior member, thereby melting the laser light absorbing member, and bonding the first exterior member and the second exterior member to each other by laser welding.
The invention is defined in the independent claims.

### Brief Description of Drawings

FIG. 1 shows the configuration of a capsule endoscope.
FIG. 2 shows the configuration of a capsule endoscope.
FIG. 3 shows the configuration of a front-end section of a capsule endoscope.
FIG. 4 shows the configuration of a front-end section of a capsule endoscope.
FIG. 5 shows the configuration of a front-end section of an endoscope.
FIG. 6 shows the configuration of a front end of an insertion section of an electronic endoscope.

### Best Mode for Carrying Out the Invention

A first example will be described below with reference to the accompanying drawings.

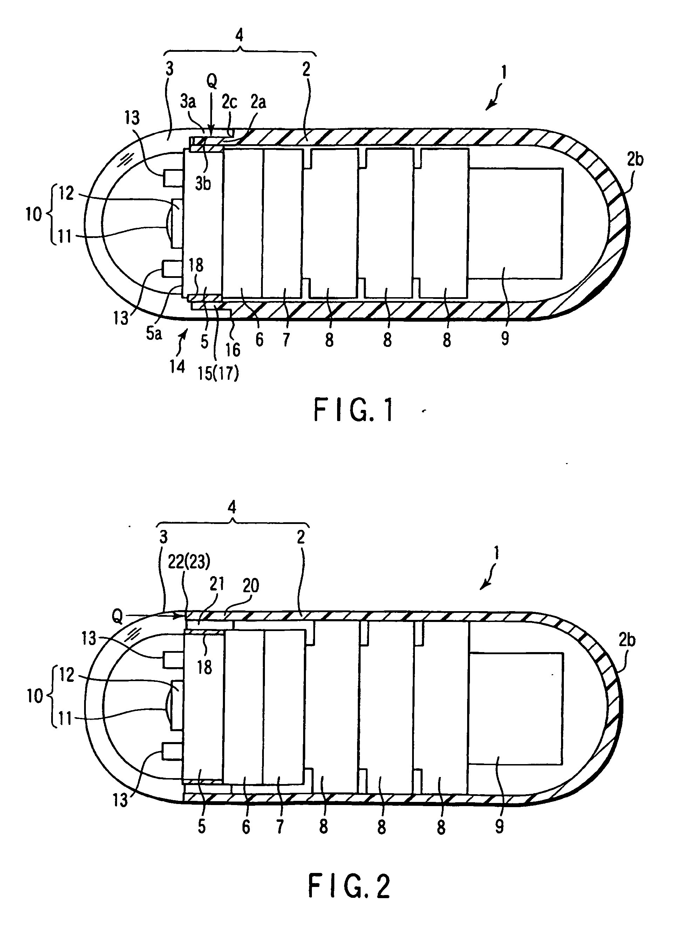

FIG. 1 shows a configuration view of a capsule endoscope. The capsule endoscope 1 is provided with a cylindrical cover 2 serving as a second exterior member, and a transparent cover 3 serving as a first exterior member. The cylindrical cover 2 and the transparent cover 3 are fitted to each other, thereby forming an integrated cylindrical capsule endoscope casing 4.

The cylindrical cover 2 is formed into a cylindrical shape. In the cylindrical cover 2, a circular second opening end section 2a is formed at one end part, and a hemispheric section 2b is formed at the other end part. The cylindrical cover 2 is formed of a resin member containing a laser light absorbing agent. That is, the cylindrical cover 2 is formed of a thermoplastic resin member which is a nontransparent resin member transmitting no laser light, and absorbs laser light (laser beam).

Types of the nontransparent resin member of the cylindrical cover 2 transmitting no laser light include, for example, polycarbonate (PC), ABC resin (ABC), polysulfone (PSU), polyphenylsulfone (PPSU), polyphenyleneoxide (PPO), polyphenylenesulfide (PPS), styrene resin, polyamide (PA) such as nylon 6 (PA6) and nylon 66 (PA66), polyethylene (PE), polypropylene (PP) and styreneacrylonitrile copolymer, and the like, each of which is mixed with a predetermined coloring agent such as carbon black. Incidentally, the nontransparent resin member may be mechanically strengthened by glass fiber or the like as the need arises.

Further, a coloring member is added to the cylindrical cover 2. As a result, when the cylindrical cover 2 is melted by irradiation of laser light, the color of the cover 2 is changed by the coloring member. Examples of the coloring member include titanium white, TiO₂, a pigment (cyanine series), and the like.

The transparent cover 3 is formed into a hemispheric shape. A circular first opening end section 3a is formed at one end part of the transparent cover 3. The transparent cover 3 is formed of an optical resin member that transmits visible light and laser light. That is, the transparent cover 3 transmits white illuminating light, and transmits, for example, reflected light from a body cavity. The transparent cover 3 is formed of a light-transmitting resin member that transmits visible light, for example, 70% or more. Further, the transparent cover 3 is formed of a member which is a light-transmitting resin member having thermoplasticity. The light-transmitting resin member of the transparent cover 3 is not particularly limited as long as it is a member that transmits laser light at a predetermined transmissivity or more.

Types of the light-transmitting resin member include, for example, polycarbonate (PC), acrylic resin, cycloolefin polymer (COP), polyurethane (PU), styrene resin, and polyamide (PA) such as nylon 6 (PA6) and nylon 66 (PA66).

In the cylindrical cover 2, a CCD imager 5 serving as an image pickup device, a signal processing circuit 6, a communication processing circuit 7, a plurality of button-shaped batteries 8, and an antenna 9 are contained. Assuming that the transparent cover 3 side of the CCD imager 5 is the front side, the signal processing circuit 6, the communication processing circuit 7, the plural button-shaped batteries 8, and the antenna 9 are provided in the order mentioned from the rear side of the CCD imager 5.

An objective optical system 10 is provided on the transparent cover 3 side of the CCD imager 5. The objective optical system 10 forms light of an optical image transmitted through the transparent cover 3 and incident thereon into an image. The objective optical system 10 includes an objective lens 11, and an optical lens arranged in a lens frame 12. The CCD imager 5 is provided at an image formation position of the objective optical system 10. Further, around the objective optical system 10, a plurality of, for example, four white LEDs 13 are provided in the same plane as an illumination optical system. Incidentally, the objective optical system 10 and the white LEDs 13 are provided in a plane 5a on the transparent cover 3 side of, for example, the CCD imager 5.

The signal processing circuit 6 includes a circuit for emission-driving the white LEDs 13, a circuit for driving the CCD imager 5, and a circuit for forming an image pickup signal output from the CCD imager 5 into an image signal.

The communication processing circuit 7 transmits an image signal formed by the signal processing circuit 6 to an external apparatus as an electric wave.

The number of button-shaped batteries provided is, for example, three. These button-shaped batteries 8 supply power to the CCD imager 5, the signal processing circuit 6, the communication processing circuit 7, and the like.

The antenna 9 is provided in a hemispheric space formed by the hemispheric section 2b of the cylindrical cover 2. The antenna 9 is electrically connected to the communication processing circuit 7, and radiates a signal processed by the communication processing circuit 7 as an electric wave.

In the cylindrical cover 2 and the transparent cover 3, the second opening end section 2a of the cylindrical cover 2 is fitted into the first opening end section 3a of the transparent cover 3, thereby integrating the cylindrical cover 2 and the transparent cover 3 with each other. The second opening end section 2a is formed by providing a step 2c on the entire circumference on the outer surface side of the cylindrical cover 2. The first opening end section 3a is formed by providing a step 3b on the entire circumference on the inner surface side of the transparent cover 3.

However, the second opening end section 2a of the cylindrical cover 2 is inserted into the first opening end section 3a of the transparent cover 3, the first opening end section 3a being on the outside, and the first opening end section 2a being on the inside, thereby achieving the fit of the covers 2 and 3. In other words, the covers 2 and 3 are in a state where the transparent cover 3 covers the outer surface of the cylindrical cover 2.

This fitting section 14 includes a boundary surface 15 at which the first opening end section 3a and the second opening end section 2a are in surface contact with each other. The boundary surface is formed concentric with the side surface of the cylindrical capsule endoscope casing 4. Incidentally, a distal end part of the first opening end section 3a is hit against the step 2c of the cylindrical cover 2, and forms a hitting surface 16.

A laser welding section 17 is formed on the entire circumference of the cylindrical boundary surface 15. At the laser welding section 17, the second opening end section 2a and the first opening end section 3a are tightly and integrally bonded to each other in a watertight state by the welding caused by external irradiation of laser light Q.

That is, the laser light Q is transmitted through the first opening end section 3a of the transparent cover 3, and is applied to the second opening end section 2a of the cylindrical cover 2 formed of the thermoplastic resin member. The irradiation direction of the laser light Q is a direction substantially perpendicular to the transparent cover 3, i.e., a direction substantially perpendicular to the side surface of the capsule endoscope casing 4. In this case, the laser light Q is applied to the entire circumference of the side surface of the capsule endoscope casing 4 while the capsule endoscope casing 4 is rotated. Alternatively, the laser light Q is applied to the entire circumference of the side surface of the capsule endoscope casing 4 while the irradiation direction of the laser light Q is changed.

The laser light Q is transmitted through the transparent cover 3, and is applied to the cylindrical cover 2. As a result of this, the second opening end section 2a of the cylindrical cover 2 is heated and melted. Thus, the second opening end section 2a and the first opening end section 3a are thermally welded, i.e., the transparent cover 3 and the cylindrical cover 2 are welded together.

The laser light Q has such a wavelength that a predetermined transmissivity or more, for example, 26% or more can be obtained with respect to the transparent cover 3. As a result of this, when the laser light Q is transmitted through the transparent cover 3 formed of the light-transmitting resin member, the energy loss of the laser light Q is reduced.

As a result, the boundary surface 15 between the first opening end section 3a formed of the light-transmitting resin member and the second opening end section 2a formed of the thermoplastic resin member is irradiated with laser light Q with less energy loss. By the irradiation of the laser light Q, energy sufficient to heat and melt the boundary surface 15 is accumulated in the boundary surface 15. Thus, sufficient heating and melting take place at the boundary surface 15 and, thereafter the first opening end section 3a and the first opening end section 2a are welded together. That is, the cylindrical cover 2 and the transparent cover 3 are welded together by the laser welding section 17.

Further, a coloring member is added to the cylindrical cover 2. As a result, when the cylindrical cover 2 is melted by the irradiation of laser light, the color of the cover 2 is changed by the coloring member. However, when the cylindrical cover 2 and the transparent cover 3 are welded together by the laser welding section 17, the fact that the welding has been achieved can be confirmed by the change in color appearing on the cylindrical cover 2.

A protection member 18 is provided at the fitting section at which the second opening end section 2a is fitted into the first opening end section 3a. More specifically, the protection member 18 is provided on the entire circumference of the inner circumferential surface of the second opening end section 2a. The protection member 18 is provided on the entire circumference of the outer circumferential surface of the CCD imager 5. Incidentally, it is sufficient if the protection member 18 is provided on one of or both of the entire circumference of the inner circumferential surface of the first opening end section 2a, and the entire circumference of the outer circumferential surface of the CCD imager 5.

The protection member 18 absorbs the laser light Q applied to the boundary surface 15 between the first opening end section 3a and the second opening end section 2a. The protection member 18 absorbs scattered light produced when the laser light Q is applied. The protection member 18 is formed of, for example, a member such as ceramic excellent in heat insulating properties and light blocking effect. As described above, the protection member 18 absorbs the laser light Q and the scattered light thereof, and hence the CCD imager 5 and the like are not adversely affected by the laser light Q and the scattered light thereof.

As described above, the capsule endoscope 1 includes the cylindrical cover 2 in which the CCD imager 5 is contained, and the transparent cover 3 which is formed of the member transmitting laser light, and through which visible light from the subject is made incident on the CCD imager 5, and the cylindrical cover 2 and the transparent cover 3 are welded together by irradiating the fitting section 14 at which the cylindrical cover 2 is fitted into the transparent cover 3 with the laser light Q from the outside of the capsule endoscope casing 4. As a result of this, even in a small-sized capsule endoscope, the cylindrical cover 2 and the transparent cover 3 can be reliably welded together, and no uneven part or burr-like part is formed on the outer surface of the capsule endoscope casing 4. The outer surface of the capsule endoscope casing 4 can therefore be maintained smooth. Furthermore, complicated post-treatment process work need not be performed.

The protection member 18 absorbs the laser light Q and the scattered light thereof. As a result of this, the laser light Q and the scattered light do not adversely affect the CCD imager 5 and the like.

Incidentally, the first example may be modified as follows. In the first example, as described above, the cylindrical cover 2 is formed of a resin member containing a laser light absorbing agent. That is, the cylindrical cover 2 is formed of a thermoplastic resin member, which is a nontransparent resin member that does not transmit laser light, and absorbs laser light. Further, when the laser light Q is transmitted through the transparent cover 3, and applied to the cylindrical cover 2, the cylindrical cover 2 is heated and melted, and the transparent cover 3 and the cylindrical cover 2 are welded together. However, the present example is not limited to this. According to the invention, a laser light absorbing member is provided at the part at which the cylindrical cover 2 and the transparent cover 3 are in surface contact with each other, and laser light Q may be applied through the transparent cover 3 to the laser light absorbing member, thereby melting the laser light absorbing member, and bonding the cylindrical cover 2 and the transparent cover 3 to each other by laser welding. The laser light absorbing member is formed of a thermoplastic resin member or an application agent that absorbs laser light. In this case, the cylindrical cover 2 need not contain a laser light absorbing agent.

Next, a second example will be described below with reference to the accompanying drawings. Incidentally, the same parts as those in FIG. 1 will be denoted by the same reference symbols and detailed description of them will be omitted.

FIG. 2 shows a configuration view of a capsule endoscope. A cylindrical cover 2 and a transparent cover 3 are made integral with each other by fitting a first opening end section 21 of the transparent cover 3 into a second opening end section 20 of the cylindrical cover 2. The second opening end section 20 is formed into a cylindrical shape. The first opening end section 21 is formed by providing a step 22 on the entire circumference on the outer surface side of the transparent cover 3.

However, the first opening end section 21 of the transparent cover 3 is inserted into the second opening end section 20 of the cylindrical cover 2, the second opening end section 20 being on the outside, and the first opening end section 21 being on the inside, thereby achieving the fit of the covers 2 and 3. In other words, the covers 2 and 3 are in a state where the cylindrical cover 2 covers the outer surface of the transparent cover 3. Further, a distal end part of the second opening end section 20 of the cylindrical cover 2 is in a state where it is hit against the step 22 of the transparent cover 3.

A laser welding section 23 is formed at a part at which the distal end part of the second opening end section 20 is hit against the step 22 of the transparent cover 3. That is, laser light Q is applied to the part at which the distal end part of the second opening end section 20 is hit against the step 22 of the transparent cover 3 in the same direction as the side surface direction of the capsule endoscope casing 4. In this case too, the laser light Q is applied to the circumferential hitting part while the capsule endoscope casing 4 is rotated. Alternatively, the laser light Q is applied to the circumferential hitting part while the irradiation position of the laser light Q is changed.

When the laser light Q is transmitted through the transparent cover 3, and is applied to the cylindrical cover 2, the distal end part of the second opening end section 20 of the cylindrical cover 2 is heated and melted. As a result of this, the transparent cover 3 and the cylindrical cover 2 are welded together.

As described above, according to the second example, even when the laser welding section 23 is formed at the part at which the distal end part of the second opening end section 20 is hit against the step 22 of the transparent cover 3, the same effect as the first embodiment can be obtained. That is, even in a small-sized capsule endoscope, the cylindrical cover 2 and the transparent cover 3 can be reliably welded together, and an uneven part or a burr-like part is not formed on the outer surface of the capsule endoscope casing 4. The outer surface of the capsule endoscope casing 4 can therefore be maintained smooth. Furthermore, complicated post-treatment process work need not be performed.

According to the invention, a laser light absorbing member is provided at the part at which the cylindrical cover 2 and the transparent cover 3 are in surface contact with each other, and laser light Q may be applied to the cylindrical cover 2, thereby melting the laser light absorbing member, and bonding the cylindrical cover 2 and the transparent cover 3 to each other by laser welding. The laser light absorbing member is formed of a thermoplastic resin member or an application agent that absorbs laser light. In this case, the cylindrical cover 2 need not contain a laser light absorbing agent.

Next, a third example will be described below with reference to the accompanying drawings.

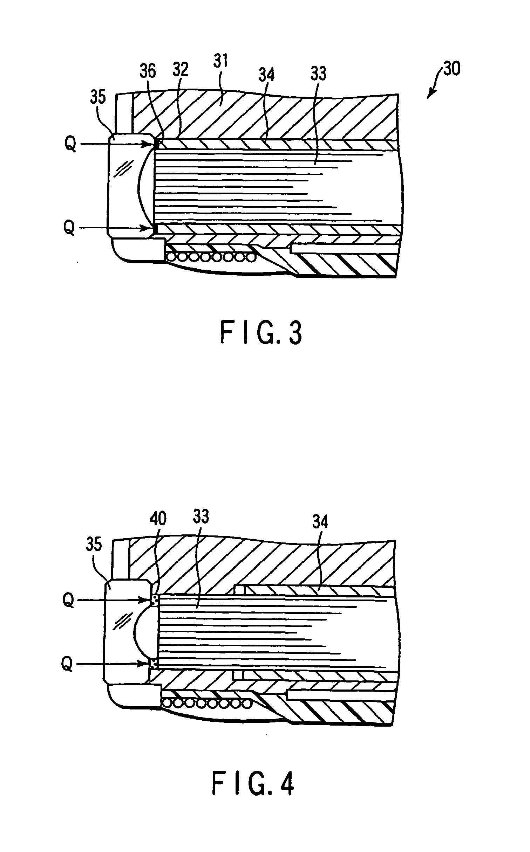

FIG. 3 shows a configuration view of an endoscope distal end section of a capsule endoscope. The endoscope distal end section 30 is a light guide part of the endoscope. The endoscope distal end section 30 is provided with a distal end constituent member 31 made of metal. The distal end constituent member 31 is provided with a hole 32 for attaching an illumination optical system. A light guide fiber bundle 33 is inserted in the hole 32 for attaching an illumination optical system, and fixed thereto. The light guide fiber bundle 33 is formed of a glass member. A pipe sleeve 34 is fixed to the light guide fiber bundle 33. The pipe sleeve 34 is formed of a thermoplastic resin member that absorbs laser light Q.

A cover glass 35 is inserted in a front end part of the hole 32 for attaching an illumination optical system. The cover glass 35 abuts on the pipe sleeve 34 at its distal end in a state where it is inserted in the hole 32 for attaching an illumination optical system. The cover glass 35 is formed of a light-transmitting resin member that transmits visible light and laser light Q.

A laser welding section 36 is formed at a part at which the cover glass 35 abuts on the pipe sleeve 34. The laser welding section 36 tightly and integrally bonds the cover glass 35 and the pipe sleeve 34 to each other, and fixes them to each other by welding caused by being irradiated with the laser light Q from outside. That is, the laser light Q is applied from the outside of the cover glass 35. The laser light Q is transmitted through the cover glass 35, and is applied to the abutment part between the cover glass 35 and the pipe sleeve 34. As a result of this, the cover glass 35 and the pipe sleeve 34 are heated and melted, thereby welding the cover glass 35 and the pipe sleeve together.

As described above, according to the third example, the laser light Q is transmitted through the cover glass 35, and is applied to the abutment part between the cover glass 35 and the pipe sleeve 34. As a result of this, the cover glass 35 and the pipe sleeve 34 are heated and melted, thereby welding the cover glass 35 and the pipe sleeve 34 together. Thus, even in a small-sized endoscope distal end section, the cover glass 35 and the pipe sleeve 34 can be reliably welded together.

Next, a fourth example will be described below with reference to the accompanying drawings. Incidentally, the same parts as those in FIG. 3 will be denoted by the same reference symbols and detailed description of them will be omitted.

FIG. 4 shows a configuration view of a capsule endoscope distal end section. A cover glass 35 is formed of a glass member. The cover glass 35 transmits visible light and laser light Q. A laser absorbing member 40 is provided between the cover glass 35 and a light guide fiber bundle 33. The laser absorbing member 40 is formed of a thermoplastic resin that absorbs laser light Q. Incidentally, a surface of the cover glass 35 in contact with the laser absorbing member 40 may be formed into, for example, an aventurine surface having a plurality of small spots.

Laser light Q is applied to the cover glass 35 from the outside thereof. The laser light Q is transmitted through the cover glass 35, and is applied to the laser absorbing member 40. As a result of this, the laser absorbing member 40 is heated and melted, thereby welding the cover glass 35 and the light guide fiber bundle 33 together. Incidentally, if the surface of the cover glass 35 in contact with the laser absorbing member 40 is formed into, for example, an aventurine surface having a plurality of small spots, the bond between the cover glass 35 and the light guide fiber bundle 33 becomes firm.
As described above, according to the fourth example, the laser absorbing member 40 is provided between the cover glass 35 and the light guide fiber bundle 33, the laser light Q is applied to the laser absorbing member 40, thereby heating and melting the laser absorbing member 40, and welding the cover glass 35 and the light guide fiber bundle 33 together. As a result of this, even in a small-sized endoscope distal end section, the cover glass 35 and the pipe sleeve 34 can be reliably welded together.

Next, a fifth example will be described below with reference to the accompanying drawings. Incidentally, the same parts as those in FIG. 3 will be denoted by the same reference symbols and detailed description of them will be omitted.

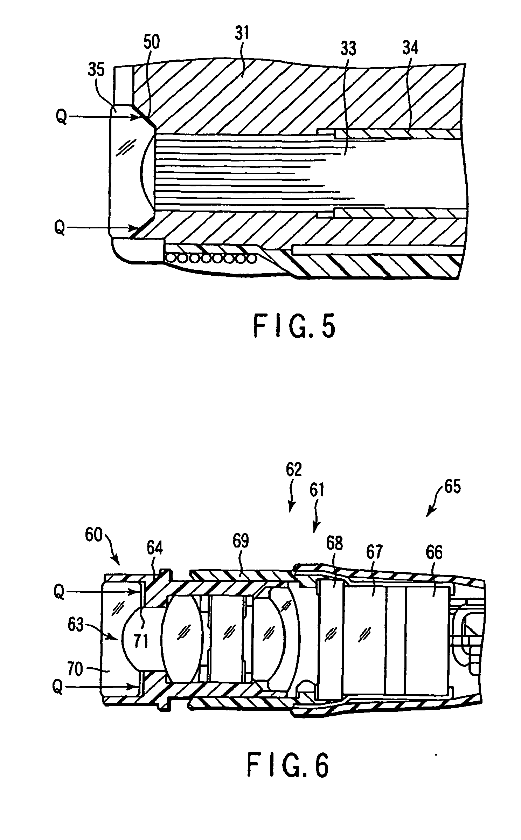

FIG. 5 shows a configuration view of a capsule endoscope distal end section. A distal end constituent member 31 is formed of a thermoplastic resin member that absorbs laser light Q. The distal end constituent member 31 is provided with a tapered opening section 50. The tapered opening section 50 is formed into a tapered shape that spreads out from the side on which the light guide fiber bundle 33 is provided toward the opened side. A caliber of the tapered opening section 50 is formed larger than a diameter of the light guide fiber bundle 33.
A cover glass 35 is provided in the tapered opening section 50. The cover glass 35 can have a diameter larger than that of the cover glass 35 used in FIG. 3 or 4. A surface of the cover glass 35 in contact with the tapered opening section 50 is formed into an inclined (tapered) surface that can be fitted in the tapered shape of the tapered opening section 50. The cover glass 35 is formed of a light-transmitting resin member that transmits visible light and laser light.

When laser light Q is applied from the outside of the cover glass 35, the laser light Q is transmitted through the cover glass 35, and is applied to an abutment part between the cover glass 35 and the tapered opening section 50 of the distal end constituent member 31. As a result of this, the cover glass 35 and the tapered opening section 50 are heated and melted, thereby welding the cover glass 35 and the tapered opening section 50 together.

As described above, according to the fifth embodiment, the distal end constituent member 31 is provided with the tapered opening section 50, and the cover glass 35 is welded to the tapered opening section 50. As a result of this, even in a small-sized endoscope distal end section, the cover glass 35 can be reliably welded to the tapered opening section 50.

Incidentally, the third to fifth examples described above are not limited to the light guide part of the endoscope, and can also be applied to an image guide.

Next, an example useful for understanding the present invention will be described below with reference to the accompanying drawings.

FIG. 6 shows a configuration view of an insertion section distal end of an electronic endoscope. The electronic endoscope insertion section distal end is constituted of a distal end constituent section formed of a hard member and a distal end insulating cover for covering the distal end constituent section. Of these members, the distal end constituent section is formed of a metallic member or the like, and is formed into a substantially pillar-like shape.

The distal end constituent section includes an imaging unit formed by integrating a first unit 60 and a second unit 61 into one unit. In the first unit 60, an objective lens 63 which is arranged at the front end, and is constituted of a plurality of optical systems is arranged in a lens frame 64 which is a first frame body, whereby an objective unit is formed.

The second unit 61 is provided behind the first unit 60. The second unit 61 includes an imaging section 65 for imaging an image made incident thereon through the objective unit. The imaging section 65 is formed by arranging a CCD chip 66, a first cover glass 67, a second cover glass 68, and the like. In the second unit 61, a channel hole or the like for arranging a forceps channel member is formed. The forceps channel member constitutes a unit insertion hole, and treatment tool insertion channel. In the unit insertion hole, the imaging unit 62 is arranged. In the treatment tool insertion channel, a treatment tool such as biopsy forceps or the like is inserted.

The second unit 61 includes a CCD holder 69 which is a second frame body. The CCD holder 69 is fitted on the rear side of the lens frame 64 of the first unit 60. The CCD holder 69 is provided on a front end surface of the imaging section 65. By fitting the CCD holder 69 on the lens frame 64, the first unit 60 and the second unit 61 are integrated into one unit, i.e., the imaging unit 62.

The objective lens 63 includes a lens 70. The lens 70 is formed of a resin member. The lens frame 64 is formed of a resin member. Accordingly, both the lens 70 and the lens frame 64 are constituted of a combination of resin members.

A resin member 71, which is a laser light absorbing member, is provided between the lens 70 and the lens frame 64.

When laser light Q is applied from the outside of the objective lens 63, the laser light Q is transmitted through the lens 70 of the objective lens 63, and is applied to the resin member 71 provided between the lens 70 and the lens frame 64. As a result of this, the lens 70 and the lens frame 64 are welded together by the melted resin member 71.

Incidentally, each of a surface of the lens 70 and a surface of the lens frame 64 which are in contact with the resin member 71 may be formed into, for example, an aventurine surface having a plurality of small spots. This makes the bond between the lens 70 and the lens frame 64 firm.

As described above, according to the sixth example, the resin member 71 is provided between the lens 70 and the lens frame 64, the resin member 71 is irradiated with the laser light Q, thereby welding the lens 70 and the lens frame 64 together by the thus melted resin member 71. As a result of this, even in a small-sized insertion section distal end of an electronic endoscope, the lens 70 and the lens frame 64 can be reliably welded together.

The above description has been given of an example in which the resin member 71 is provided and, if the lens frame 64 is formed of a laser light absorbing member, the resin member 71 may be omitted.

## Claims

1. A capsule endoscope (1) comprising a first exterior member (3) and a second exterior member (2), wherein:
the first exterior member (3) is formed of an optical resin member capable of transmitting visible light and laser light (Q) having a wavelength suitable to obtain a predetermined transmissivity or more with respect to the first exterior member (2);
the second exterior member (2) is arranged with respect to the first exterior member (3) in such a manner that the second exterior member (2) is brought into surface contact with the first exterior member (3), and is formed of a resin member,
**characterized by**
a laser light absorbing member formed of a thermoplastic resin member or an application agent, the laser light absorbing member being provided at a part at which the first exterior member (3) and the second exterior member (2) are in surface contact with each other,
the laser light absorbing member being capable of absorbing said laser light being applied to the laser light absorbing member through the first exterior member (3), thereby melting the laser light absorbing member, and the first exterior member (3) and the second exterior member (2) are bonded to each other by laser welding.

2. The capsule endoscope according to claim 1, **characterized in that** a capsule endoscope casing (4) formed by bonding the first exterior member (3) and the second exterior member (2) to each other is formed into a cylindrical shape, and
a bonded part at which the first exterior member (3) and the second exterior member (2) are bonded to each other is provided on the entire circumference of a side surface of the capsule endoscope (1).

3. The capsule endoscope according to claim 1, **characterized in that** in a bonded part at which the first exterior member (3) and the second exterior member (2) are bonded to each other, the second exterior member (2) is fitted into the first exterior member (3), wherein the first exterior member (3) covers the outer surface of the second exterior member (2).

4. The capsule endoscope according to claim 1, further comprising:
a first opening end section (3a) formed in the first exterior member (3); and
a second opening end section (2a) formed in the second exterior member (2), **characterized in that**
in a bonded part at which the first exterior member (3) and the second exterior member (2) are bonded to each other, the second opening end section (2a) is inserted/fitted into the first opening end section (3a), and at a boundary surface between the first opening end section (3a) and the second opening end section (2a), the first opening end section (3a) and the second opening end section (2a) are integrally and tightly fixed to each other by welding achieved by laser light irradiation.

5. The capsule endoscope according to claim 4, **characterized in that**
the bonded part at which the first exterior member (3) and the second exterior member (2) are bonded to each other includes a fitting surface (15) at which the first opening end section (3a) and the second opening end section (2a) are in surface contact with each other, and a hitting surface (16) at which the first opening end section (3a) and the second opening end section (2a) hit against each other, and is formed by welding achieved by irradiating at least one of the fitting surface (15) and the hitting surface with the laser light (Q).

6. The capsule endoscope according to claim 1, **characterized in that**
the second exterior member (2) is formed of a thermoplastic resin member.

7. The capsule endoscope according to claim 1, **characterized in that**
the second exterior member (2) contains a coloring member.

8. The capsule endoscope according to claim 1, **characterized in that**
the laser light absorbing member is provided on an inner wall surface of the first exterior member (3).

9. The capsule endoscope according to claim 1, **characterized in that**
the laser light absorbing member is provided on an outer wall surface of the second exterior member (2).

10. The capsule endoscope according to claim 1, **characterized in that**
the laser light (Q) is provided with a transmissivity equal to 26% or more with respect to the first exterior member (3).

11. The capsule endoscope according to claim 1, **characterized by**
further comprising a protection member (18) which is provided at a fitting section at which the end section of the second exterior member (2) is fitted into the end section of the first exterior member (3), and absorbs at least the laser light (Q).

12. The capsule endoscope according to claim 2, **characterized by**
further comprising a protection member (18) which is provided at a fitting section at which the end section of the second exterior member (2) is fitted into the end section of the first exterior member (3), and absorbs at least the laser light (Q).

13. A method of manufacturing a capsule endoscope (1) comprising:
arranging a second exterior member (2) formed of a resin member with respect to a first exterior member (3) formed of an optical resin member in such a manner that the second exterior member (2) is brought into surface contact with the first exterior member (3), wherein the first exterior member (3) is formed of an optical resin member adapted for transmitting visible light and laser light (Q) having a wavelength suitable to obtain a predetermined transmissivity or more with respect to the first exterior member (2),
**characterized by** comprising:
providing a laser light absorbing member at a part at which the first exterior member (3) and the second exterior member (2) are in surface contact with each other, the laser light absorbing member being formed of a thermoplastic resin member or an application agent and being capable of absorbing said laser light; and
applying said laser light (Q) to the laser light absorbing member through the first exterior member (3), thereby melting the laser light absorbing member, and bonding the first exterior member (3) and the second exterior member (2) to each other by laser welding.

## Patentansprüche

1. Kapselendoskop (1), das ein erstes externes Element (3) und ein zweites externes Element (2) umfasst, wobei:
das erste externe Element (3) aus einem optischen Harzelement gebildet ist, das dazu in der Lage ist, sichtbares Licht und Laserlicht (Q) zu transmittieren, das eine geeignete Wellenlänge hat, um eine vorbestimmte Transmissivität oder mehr bezüglich des ersten externen Elements (2) zu erhalten;
das zweite externe Element (2) bezüglich des ersten externen Elements (3) so angeordnet ist, dass das zweite externe Element (2) mit dem ersten externen Element (3) in Oberflächenkontakt gebracht ist, und aus einem Harzelement gebildet ist,
**gekennzeichnet durch**
ein Laserlicht absorbierendes Element, das aus einem thermoplastischen Harzelement oder einem Applikationsmittel gebildet ist, wobei das Laserlicht absorbierende Element an einen Teil vorgesehen ist, an dem das erste externe Element (3) und das zweite externe Element (2) in Oberflächenkontakt miteinander stehen, und wobei
das Laserlicht absorbierende Element dazu in der Lage ist, das Laserlicht zu absorbieren, das **durch** das erste externe Element (3) auf das Laserlicht absorbierende Element aufgebracht wird, wodurch das Laserlicht absorbierende Element schmilzt und das erste externe Element (3) und das zweite externe Element (2) **durch** Laserschweißen miteinander verbindet.

2. Kapselendoskop gemäß Anspruch 1, **dadurch gekennzeichnet, dass**
ein Kapselendoskopgehäuse (4), das durch Verbinden des ersten externen Elements (3) mit dem zweiten externen Element (2) miteinander gebildet ist, in einer zylindrischen Form ausgebildet ist, und
ein verbundener Teil, an dem das erste externe Element (3) und das zweite externe Element (2) miteinander verbunden sind, an dem gesamten Umfang einer Seitenoberfläche des Kapselendoskops (1) vorgesehen ist.

3. Kapselendoskop gemäß Anspruch 1, **dadurch gekennzeichnet, dass** in einem verbundenen Teil, an dem das erste externe Element (3) und das zweite externe Element (2) miteinander verbunden sind, das zweite externe Element (2) in das erste externe Element (3) eingepasst ist, wobei das erste externe Element (3) die äußere Oberfläche des zweiten externen Elements (2) bedeckt.

4. Kapselendoskop gemäß Anspruch 1, das ferner umfasst:
einen ersten Öffnungsendabschnitt (3a), der in dem ersten externen Element (3) gebildet ist; und
einen zweiten Öffnungsendabschnitt (2a), der in dem zweiten externen Element (2) gebildet ist,
**dadurch gekennzeichnet, dass**
in einem verbundenen Teil, an dem das erste externe Element (3) und das zweite externe Element (2) miteinander verbunden sind, der zweite Öffnungsendabschnitt (2a) in den ersten Öffnungsendabschnitt (3a) eingeführt/eingepasst ist und an einer Grenzoberfläche zwischen dem ersten Öffnungsendabschnitt (3a) und dem zweiten Öffnungsendabschnitt (2a) der erste Öffnungsendabschnitt (3a) und der zweite Öffnungsendabschnitt (2a) durch Schweißen, das durch Laserlichtbestrahlung erreicht wird, integriert und fest aneinander fixiert sind.

5. Kapselendoskop gemäß Anspruch 4, **dadurch gekennzeichnet, dass**
der verbundene Teil, an dem das erste externe Element (3) und das zweite externe Element (2) miteinander verbunden sind, eine Einpassoberfläche (15) umfasst, an der der erste Öffnungsendabschnitt (3a) und der zweite Öffnungsendabschnitt (2a) in Oberflächenkontakt miteinander stehen und eine Auftreffoberfläche (16), an der der erste Öffnungsendabschnitt (3a) und der zweite Öffnungsendabschnitt (2a) aufeinander treffen, durch Schweißen gebildet ist, das durch Laserlichtbestrahlung der Einpassoberfläche (15) und/oder der Auftreffoberfläche (16) mit dem Laserlicht (Q) erreicht wird.

6. Kapselendoskop gemäß Anspruch 1, **dadurch gekennzeichnet, dass**
das zweite externe Element (2) aus einem thermoplastischen Harzelement gebildet ist.

7. Kapselendoskop gemäß Anspruch 1, **dadurch gekennzeichnet, dass**
das zweite externe Element (2) ein Farbelement enthält.

8. Kapselendoskop gemäß Anspruch 1, **dadurch gekennzeichnet, dass**
das Laserlicht absorbierende Element an einer inneren Wandoberfläche des ersten externen Elements (3) vorgesehen ist.

9. Kapselendoskop gemäß Anspruch 1, **dadurch gekennzeichnet, dass**
das Laserlicht absorbierende Element an einer äußeren Wandoberfläche des zweiten externen Elements (2) vorgesehen ist.

10. Kapselendoskop gemäß Anspruch 1, **dadurch gekennzeichnet, dass**
das Laserlicht (Q) mit einer Transmissivität von 26 % oder mehr bezüglich des ersten externen Elements (3) vorgesehen ist.

11. Kapselendoskop gemäß Anspruch 1, **dadurch gekennzeichnet, dass**
es ferner ein Schutzelement (18) umfasst, das an einem Einpassabschnitt vorgesehen ist, an dem der Endabschnitt des zweiten externen Elements (2) in den Endabschnitt des ersten externen Elements (3) eingepasst ist, und wenigstens das Laserlicht (Q) absorbiert.

12. Kapselendoskop gemäß Anspruch 2, **dadurch gekennzeichnet**, das
es ferner ein Schutzelement (18) umfasst, das an einem Einpassabschnitt vorgesehen ist, an dem der Endabschnitt des zweiten externen Elements (2) in den Endabschnitt des ersten externen Elements (3) eingepasst ist, und wenigstens das Laserlicht (Q) absorbiert.

13. Verfahren zur Herstellung eines Kapselendoskops (1), das umfasst:
Anordnen eines zweiten externen Elements (2), das aus einem Harzelement gebildet ist, bezüglich eines ersten externen Elements (3), das aus einem optischen Harzelement gebildet ist, in einer derartigen Art und Weise, dass das zweite externe Element (2) mit dem ersten externen Element (3) in Oberflächenkontakt gebracht wird, wobei das erste externe Element (3) aus einem optischen Harzelement gebildet ist, das dazu eingerichtet ist, sichtbares Licht und Laserlicht (Q) zu transmittieren, das eine geeignete Wellenlänge hat, um eine vorbestimmte Transmissivität oder mehr bezüglich des ersten externen Elements (2) zu erhalten,
**dadurch gekennzeichnet, dass** es umfasst:
Bereitstellen eines Laserlicht absorbierenden Elements an einem Teil, an dem das erste externe Element (3) und das zweite externe Element (2) in Oberflächenkontakt miteinander stehen, wobei das Laserlicht absorbierende Element aus einem thermoplastischen Harzelement oder einem Applikationsmittel gebildet ist und dazu in der Lage ist, das Laserlicht zu absorbieren; und
Aufbringen des Laserlichts (Q) auf das Laserlicht absorbierende Element durch das erste externe Element (3), wodurch das Laserlicht absorbierende Element schmilzt und das erste externe Element (3) und das zweite externe Element (2) durch Laserschweißen miteinander verbunden werden.

## Revendications

1. Endoscope (1) de type capsule comprenant un premier élément extérieur (3) et un deuxième élément extérieur (2), dans lequel :
le premier élément extérieur (3) est formé d'un élément en résine optique capable de transmettre une lumière visible et une lumière laser (Q) présentant une longueur d'onde appropriée pour obtenir une transmissivité prédéterminée ou plus par rapport au premier élément extérieur (2) ;
le deuxième élément extérieur (2) est agencé par rapport au premier élément extérieur (3) d'une manière telle que le deuxième élément extérieur (2) est amené en contact de surface avec le premier élément extérieur (3), et est formé d'un élément en résine,
**caractérisé par**
un élément d'absorption de lumière laser formé d'un élément en résine thermoplastique ou d'un agent d'application, l'élément d'absorption de lumière laser étant prévu au niveau d'une partie au niveau de laquelle le premier élément extérieur (3) et le deuxième élément extérieur (2) sont en contact de surface l'un avec l'autre,
l'élément d'absorption de lumière laser étant capable d'absorber ladite lumière laser étant appliquée sur l'élément d'absorption de lumière laser par l'intermédiaire du premier élément extérieur (3), faisant fondre de ce fait l'élément d'absorption de lumière laser, et collant le premier élément extérieur (3) et le deuxième élément extérieur (2) l'un à l'autre par soudage laser.

2. Endoscope de type capsule selon la revendication 1, **caractérisé en ce que**
un boîtier (4) d'endoscope de type capsule formé en collant le premier élément extérieur (3) et le deuxième élément extérieur (2) l'un à l'autre est formé selon une forme cylindrique, et
une partie collée au niveau de laquelle le premier élément extérieur (3) et le deuxième élément extérieur (2) sont collés l'un à l'autre est prévue sur la circonférence entière d'une surface latérale de l'endoscope (1) de type capsule.

3. Endoscope de type capsule selon la revendication 1, **caractérisé en ce que** dans une partie collée au niveau de laquelle le premier élément extérieur (3) et le deuxième élément extérieur (2) sont collés l'un à l'autre, le deuxième élément extérieur (2) est ajusté dans le premier élément extérieur (3), le premier élément extérieur (3) cependant couvrant la surface externe du deuxième élément extérieur (2).

4. Endoscope de type capsule selon la revendication 1, comprenant en outre :
une première section (3a) d'extrémité d'ouverture formée dans le premier élément extérieur (3) ; et
une deuxième section (2a) d'extrémité d'ouverture formée dans le deuxième élément extérieur (2),
**caractérisé en ce que**
dans une partie collée au niveau de laquelle le premier élément extérieur (3) et le deuxième élément extérieur (2) sont collés l'un à l'autre, la deuxième section (2a) d'extrémité d'ouverture est insérée/ajustée dans la première section (3a) d'extrémité d'ouverture, et au niveau d'une surface frontière entre la première section (3a) d'extrémité d'ouverture et la deuxième section (2a) d'extrémité d'ouverture, la première section (3a) d'extrémité d'ouverture et la deuxième section (2a) d'extrémité d'ouverture sont fixées solidairement et solidement l'une à l'autre par soudage obtenu par irradiation d'une lumière laser.

5. Endoscope de type capsule selon la revendication 4, **caractérisé en ce que**
la partie collée au niveau de laquelle le premier élément extérieur (3) et le deuxième élément extérieur (2) sont collés l'un à l'autre comprend une surface d'ajustement (15) au niveau de laquelle la première section (3a) d'extrémité d'ouverture et la deuxième section (2a) d'extrémité d'ouverture sont en contact de surface l'une avec l'autre, et une surface de choc (16) au niveau de laquelle la première section (3a) d'extrémité d'ouverture et la deuxième section (2a) d'extrémité d'ouverture se heurtent l'une contre l'autre, et est formée par soudage obtenu en irradiant au moins l'une parmi la surface d'ajustement (15) et la surface de choc avec la lumière laser (Q).

6. Endoscope de type capsule selon la revendication 1, **caractérisé en ce que**
le deuxième élément extérieur (2) est formé d'un élément en résine thermoplastique.

7. Endoscope de type capsule selon la revendication 1, **caractérisé en ce que**
le deuxième élément extérieur (2) contient un élément de coloration.

8. Endoscope de type capsule selon la revendication 1, **caractérisé en ce que**
l'élément d'absorption de lumière laser est prévu sur une surface de paroi interne du premier élément extérieur (3).

9. Endoscope de type capsule selon la revendication 1, **caractérisé en ce que**
l'élément d'absorption de lumière laser est prévu sur une surface de paroi externe du deuxième élément extérieur (2).

10. Endoscope de type capsule selon la revendication 1, **caractérisé en ce que**
la lumière laser (Q) est délivrée avec une transmissivité égale à 26 % ou plus par rapport au premier élément extérieur (3).

11. Endoscope de type capsule selon la revendication 1, **caractérisé en ce qu'**il comprend en outre
un élément de protection (18) qui est prévu au niveau d'une section d'ajustement au niveau de laquelle la section d'extrémité du deuxième élément extérieur (2) est ajusté dans la section d'extrémité du premier élément extérieur (3), et absorbe au moins la lumière laser (Q).

12. Endoscope de type capsule selon la revendication 2, **caractérisé en ce qu'**il comprend en outre
un élément de protection (18) qui est prévu au niveau d'une section d'ajustement au niveau de laquelle la section d'extrémité du deuxième élément extérieur (2) est ajusté dans la section d'extrémité du premier élément extérieur (3), et absorbe au moins la lumière laser (Q).

13. Procédé de fabrication d'un endoscope (1) de type capsule comprenant l'étape consistant à :
agencer un deuxième élément extérieur (2) formé d'un élément en résine par rapport à un premier élément extérieur (3) formé d'un élément en résine optique d'une manière telle que le deuxième élément extérieur (2) est amené en contact de surface avec le premier élément extérieur (3), dans lequel le premier élément extérieur (3) est formé d'un élément en résine optique adapté pour transmettre la lumière visible et la lumière laser (Q) présentant une longueur d'onde appropriée pour obtenir une transmissivité prédéterminée ou plus par rapport au premier élément extérieur (2),
**caractérisé en ce qu'**il comprend les étapes consistant à :
prévoir un élément d'absorption de lumière laser au niveau d'une partie au niveau de laquelle le premier élément extérieur (3) et le deuxième élément extérieur (2) sont en contact de surface l'un avec l'autre, l'élément d'absorption de lumière laser étant formé d'un élément en résine thermoplastique ou d'un agent d'application et étant capable d'absorber ladite lumière laser ; et
appliquer ladite lumière laser (Q) sur l'élément d'absorption de lumière laser par l'intermédiaire du premier élément extérieur (3), faisant fondre de ce fait l'élément d'absorption de lumière laser, et collant le premier élément extérieur (3) et le deuxième élément extérieur (2) l'un à l'autre par soudage laser.
